(19) Europäisches Patentamt
European Patent Office
Office européen des brevets

(11) Veröffentlichungsnummer: **0 029 183**
**B1**

# (12) EUROPÄISCHE PATENTSCHRIFT

(45) Veröffentlichungstag der Patentschrift: **12.01.83**

(51) Int. Cl.³: **C 07 D 257/04,** C 07 D 403/12, C 07 D 401/12, C 07 D 413/12, C 07 D 453/06, A 01 N 43/64 // C07D211/16, C07D215/08, C07D209/04

(21) Anmeldenummer: **80106848.7**

(22) Anmeldetag: **06.11.80**

(54) **Tetrazolyloxycarbonsäureamide, Verfahren zu ihrer Herstellung und ihre Verwendung als Herbizide.**

(30) Priorität: **17.11.79 DE 2946432**

(43) Veröffentlichungstag der Anmeldung: **27.05.81 Patentblatt 81/21**

(45) Bekanntmachung des Hinweises auf die Patenterteilung: **12.01.83 Patentblatt 83/2**

(84) Benannte Vertragsstaaten: **AT BE CH DE FR GB IT LI NL SE**

(56) Entgegenhaltungen:
**EP-A-0 005 501**
**DE-A-2 637 886**

(73) Patentinhaber: **BAYER AG, Zentralbereich Patente, Marken und Lizenzen, D-5090 Leverkusen 1, Bayerwerk (DE)**

(72) Erfinder: **Förster, Heinz, Dr., Am Eckbusch 47, D-5600 Wuppertal 1 (DE)**
Erfinder: **Hofer, Wolfgang, Dr., Pahlkestrasse 59, D-5600 Wuppertal 1 (DE)**
Erfinder: **Maurer, Fritz, Dr., Roeberstrasse 8, D-5600 Wuppertal 1 (DE)**
Erfinder: **Mues, Volker, Dr., Gellertweg 13, D-5600 Wuppertal 1 (DE)**
Erfinder: **Eue, Ludwig, Dr., Paul-Klee-Strasse 36, D-5090 Leverkusen (DE)**
Erfinder: **Schmidt, Robert Rudolf, Dr., Hahnenweg 5, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 029 183 B1

# Tetrazolyloxycarbonsäureamide, Verfahren zu ihrer Herstellung und ihre Verwendung als Herbizide

Die Erfindung betrifft neue Tretrazolyloxycarbonsäureamide, ein Verfahren zu ihrer Herstellung und ihre Verwendung als Herbizide.

Es ist bereits bekannt geworden, dass bestimmte Phenoxycarbonsäureamide, wie z.B. 2,4-Dichlorphenoxy-essigsäureamid, herbizid wirksam sind (vergleiche FR-PS 1 313 840). Die als Herbizide bekannten Phenoxycarbonsäureamide zeigen jedoch bei den üblichen Aufwandmengen nur eine geringe Wirkung gegen Ungräser und können zur Bekämpfung von Unkräutern in verschiedenen dikotylen Kulturen wegen mangelnder Selektivität nicht verwendet werden.

Es wurden nun neue Tetrazolyloxycarbonsäureamide der Formel

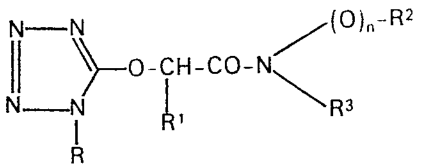

(I)

gefunden, in welcher

R für Phenyl steht, welches gegebenenfalls substituiert ist durch Chlor, Nitro, Methyl und/oder Trifluormethyl, wobei auch mehrfache und gemischte Substitutionen durch die genannten Reste möglich ist, in welcher weiter

$R^1$ für Wasserstoff steht,

n für Null oder 1 steht,

$R^2$ für $C_1$–$C_6$-Alkyl, $C_1$–$C_4$-Alkoxy-ethyl, Allyl, Propargyl, 1-Methyl-propargyl oder 1,1-Dimethylpropargyl oder – für den Fall, dass n für Null steht – auch für Cyanomethyl, Cyclopentyl, Cyclohexyl, Benzyl oder Phenyl steht,

in welcher weiter

$R^3$ für $C_1$–$C_6$-Alkyl, $C_1$–$C_4$-Alkoxy-ethyl, Allyl, Propargyl, 1-Methyl-propargyl, 1,1-Dimethyl-propargyl, Cyanomethyl, Cyclopentyl, Cyclohexyl, Benzyl, Naphtyl oder Phenyl steht, welches gegebenenfalls durch Methyl, Chlor, Cyano, Nitro oder Methoxy substituiert ist, wobei auch mehrfache und gemischte Substitution durch die genannten Reste möglich ist,

in welcher weiter – für den Fall, dass n für Null steht – die Reste $R^2$ und $R^3$ zusammen mit dem Stickstoffatom, an das sie gebunden sind, für Pyrrolidyl, Monoalkyl- oder Dialkyl-pyrrolidyl mit 1 bis 3 Kohlenstoffatomen je Alkylgruppe, Morpholinyl oder Dialkylmorpholinyl mit 1 bis 3 Kohlenstoffatomen je Alkylgruppe, Piperidyl, Monoalkyl-, Dialkyl-, oder Trialkylpiperidyl mit 1 bis 3 Kohlenstoffatomen je Alkylgruppe, für 4,4-Dialkoxy-piperidyl mit 1 bis 3 Kohlenstoffatomen je Alkoxygruppe, für spirosubstituiertes Piperidyl der Formel

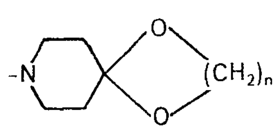

(worin n für 2 oder 3 steht), für Perhydroazepinyl (Hexamethylenimino-Rest), Trimethyl-perhydroazepinyl, für den Heptamethylenimino-Rest, für den Dodekamethylenimino-Rest, für 1,2,3,4-Tetrahydroindolyl, für Monoalkyl-, Dialkyl- oder Trialkyl-1,2,3,4-tetrahydroindolyl mit bis zu 3 Kohlenstoffatomen je Alkylgruppe, für Perhydroindolyl, Monoalkyl-, Dialkyl- oder Trialkyl-perhydroindolyl mit 1 bis 3 Kohlenstoffatomen je Alkylgruppe, 1,2,3,4-Tetrahydrochinolyl oder 1,2,3,4-Tetrahydro-iso-chinolyl, Monoalkyl-, Dialkyl- oder Trialkyl-1,2,3,4-tetrahydrochinolyl oder -isochinolyl mit 1 bis 3 Kohlenstoffatomen je Alkylgruppe, für Perhydrochinolyl oder Perhydro-iso-chinolyl, Monoalkyl-, -Dialkyl- oder Trialkylperhydrochinolyl oder -perhydroisochinolyl mit 1 bis 3 Kohlenstoffatomen je Alkylgruppe, für Perhydrothiazolyl, für Perhydrooxazolyl, für Perhydrooxazinyl, für den Rest

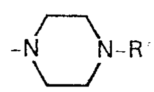

(worin R′ für $C_1$–$C_4$-Alkyl, für gegebenenfalls durch $C_1$–$C_2$-alkyl, $C_1$–$C_2$-Alkoxy, Fluor, Chlor, Brom, Trifluormethyl und/oder Nitro substituiertes Phenyl, für Benzyl oder Phenyläthyl steht) oder für den Rest

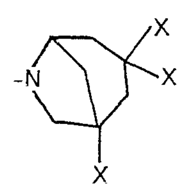

(worin X Wasserstoff oder Methyl bedeutet) stehen.

Man erhält die neuen Tetrazolyloxycarbonsäureamide der Formel (I), wenn man α-Hydroxycarbonsäureamide der Formel

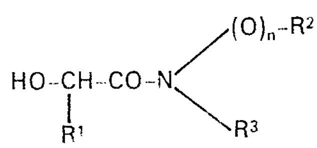

(II)

in welcher

n, $R^1$, $R^2$ und $R^3$ die oben angegebene Bedeutung haben, mit Halogentetrazolen der Formel

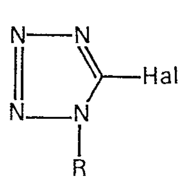

(III)

in welcher
R die oben angegebene Bedeutung hat und Hal für Chlor, Brom oder Jod steht,
gegebenenfalls in Gegenwart eines Säureakzeptors und gegebenenfalls unter Verwendung eines Verdünnungsmittels umsetzt.

Die neuen Tetrazolyloxycarbonsäureamide der Formel (I) zeichnen sich durch starke herbizide Wirksamkeit aus.

Überraschenderweise zeigen die neuen Tetrazolyloxycarbonsäureamide eine wesentlich bessere und andersartige herbizide Wirkung als die aus dem Stand der Technik bekannten Phenoxycarbonsäureamide. Insbesondere überrascht die Tatsache, dass die erfindungsgemässen Verbindungen bei guter Verträglichkeit gegenüber Nutzpflanzen neben ihrer hohen Wirkung gegen dikotyle Unkräuter auch sehr gute Wirkung gegen Ungräser zeigen, während konstitutionell ähnliche Phenoxy-alkancarbonsäurederivate, wie z.B. 2,4-Dichlorphenoxy-essigsäureamid, nur geringe Wirkung gegen Gramineen aufweisen. Sie eignen sich ausserdem zur selektiven Unkrautbekämpfung in Rüben, Sojabohnen, Baumwolle, Mais, Reis und anderen Getreidearten.

Verwendet man beispielsweise 5-Chlor-1-phenyl-(1-H)-tetrazol und Hydroxyessigsäurepiperidid als Ausgangsstoffe, so kann der Reaktionsablauf nach dem erfindungsgemässen Verfahren durch folgende Formelschemata wiedergegeben werden:

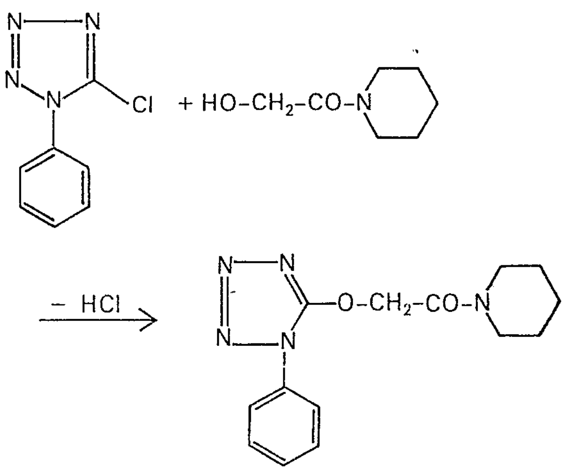


Als Ausgangsstoffe der Formel (II) seien beispielsweise die folgenden α-Hydroxy-carbonsäureamide genannt:

N-Methoxy-N-methyl-, N-Ethoxy-N-methyl-, N-n-Propoxy-N-methyl-, N-iso-Propoxy-N-methyl-, N-Ethoxy-N-ethyl-, N-n-Propoxy-N-ethyl-, N-iso-Propoxy-N-ethyl-, N-n-Propoxy-N-n-propyl-, N-iso-Propoxy-N-isopropyl-, N-iso-Propoxy-N-n-propyl-, N-Methoxy-N-ethyl-, N-Methoxy-N-n-propyl-, N-Methoxy-N-isopropyl-, N-Methoxy-N-n-butyl-, N-Methoxy-N-isobutyl-, N-Methoxy-N-sek.-butyl-, N-Methoxy-N-sek.-hexyl-, N-Ethoxy-N-n-propyl-, N-Ethoxy-N-isopropyl-, N-(2-Ethoxy-ethoxy)-N-methyl-, N-(2-Ethoxy-ethoxy)-N-ethyl-, N-(2-Ethoxy-ethoxy)-N-n-propyl, N-(2-Ethoxy-ethoxy)-N-isopropyl-, N-(2-Ethoxy-ethoxy)-N-cyclohexyl-, N-Allyloxy-N-allyl-, N-Allyloxy-N-methyl-, N-Allyloxy-N-ethyl-, N-Allyloxy-N-n-propyl-, N-Allyloxy-N-isopropyl-, N-Allyloxy-N-n-butyl-, N-Allyloxy-N-iso-butyl-, N-Allyloxy-N-sek.-butyl-, N-Methoxy-N-cyclopentyl-, N-Methoxy-N-cyclohexyl-, N-Methoxy-N-(2-ethoxy-ethyl)-, N-Ethoxy-N-(2-ethoxy-ethyl)-, N-(2-Ethoxy-ethoxy)-N-(2-ethoxy-ethyl)- und N-(2-Ethoxy-ethoxy)-N-sek.-hexyl-hydroxy-essigsäureamid,
Hydroxyessigsäuredimethylamid, -diethylamid, -di-n-propyl-amid, -di-isopropylamid, -N-methyl-N-iso-propylamid, -N-methyl-N-iso-butylamid, -N-methyl-N-sek.-butylamid, -di-(2-ethyl-hexyl)-amid, -N-methyl-N-(2-cyano-ethyl)-amid, -di-(2-methoxy-ethyl)-amid, -di-allylamid, -N-methyl-N-propargylamid, -N-methyl-N-(1-methyl-propargyl)-amid, -dipropargylamid, -N-methyl-N-cyclopentylamid, -N-methyl-N-cyclohexyl-amid, -N-methyl-anilid, -N-methyl-N-(2-nitro-phenyl)-, -N-methyl-N-(3-nitrophenyl)-, -N-methyl-N-(4-nitro-phenyl)-amid, -N-methyl-N-(2-chlorphenyl)-, -N-methyl-N-(3-chlorphenyl)-, -N-methyl-N-(4-chlor-phenyl)-amid, -N-methyl-N-(3-nitro-6-methyl-phenyl)-amid, -N-ethyl-anilid, -N-ethyl-N-(2-nitro-phenyl)-, -N-ethyl-N-(3-nitro-phenyl)-, -N-äthyl-N-(4-nitro-phenyl)-amid, -N-ethyl-N-(2-chlor-phenyl)-, -N-ethyl-N-(3-chlor-phenyl)-, -N-ethyl-N-(4-chlor-phenyl)-amid, -N-ethyl-N-(3-nitro-6-methyl-phenyl)-amid, -N-propyl-anilid, -N-propyl-N-(2-nitro-phenyl)-, -N-propyl-N-(3-nitro-phenyl)-, -N-propyl-N-(4-nitro-phenyl)-amid, -N-propyl-N-(2-chlor-phenyl)-, -N-propyl-N-(3-chlor-phenyl)-, -N-propyl-N-(4-chlor-phenyl)-amid, -N-propyl-N-(2-methyl-phenyl)-, -N-propyl-N-(3-methyl-phenyl)-, -N-propyl-N-(4-methyl-phenyl)-amid, -N-propyl-N-(3-nitro-6-methyl-phenyl)-amid, -N-butyl-anilid, -N-butyl-N-(2-nitro-phenyl)-, -N-butyl-N-(3-nitro-phenyl)-, -N-butyl-N-(4-nitro-phenyl)-amid, -N-butyl-N-(2-chlor-phenyl)-, -N-butyl-N-(3-chlor-phenyl)-, -N-butyl-N-(4-chlor-phenyl)-amid, -N-butyl-N-(2-methyl-phenyl)-, -N-butyl-N-(3-methyl-phenyl)-, -N-butyl-N-(4-methyl-phenyl)-amid, -N-butyl-N-(3-nitro-6-methyl-phenyl)-amid, N-isobutyl-anilid,

-N-iso-butyl-N-(2-nitro-phenyl)-, -N-iso-butyl-N-(3-nitro-phenyl)-, -N-iso-butyl-N-(4-nitro-phenyl)-amid, -N-iso-butyl-N-(2-chlor-phenyl)-, -N-isobutyl-N-(3-chlor-phenyl)-, -N-iso-butyl-N-(4-chlor-phenyl)-amid, -N-iso-butyl-N-(2-methyl-phenyl)-, -N-iso-butyl-N-(3-methyl-phenyl)-, -N-iso-butyl-N-(4-methyl-phenyl)-amid, -N-iso-butyl-N-(3-nitro-6-methyl-phenyl)-amid, -N-methyl-N-naphth(1)ylamid, -N-methyl-N-naphth(2)ylamid, -N-ethyl-N-naphth(1)ylamid, -N-äthyl-N-naphth(2)ylamid, -N-n-propyl-N-naphth(2)ylamid, -N-iso-propyl-N-naphth(2)ylamid, -N-n-butyl-N-naphth(2)yl-amid, -N-iso-butyl-n-naphth(2)ylamid, -dibenzylamid, -N-methyl-N-benzylamid, -N-ethyl-N-benzylamid, -N-propyl-N-benzylamid, -N-butyl-N-benzylamid, -pyrrolidid, -2-methyl-pyrrolidid, -morpholid, -3,5-dimethyl-morpholid-piperidid, -2-methyl-piperidid, -4-methyl-piperidid, -2,4-dimethyl-piperidid, -2,4,6-trimethyl-piperidid, -2-ethyl-piperidid, -4-ethyl-piperidid, -2,4-diethyl-piperidid, -2,4,6-triethyl-piperidid, -2-methyl-4-äthyl-piperidid, -2-ethyl-4-methyl-piperidid, -2-methyl-5-ethyl-piperidid, -2-ethyl-5-methyl-piperidid, -2-methyl-6-ethyl-piperidid, -1,2,3,4-tetrahydroindolid, -2-methyl-1,2,3,4-tetrahydrochinolid, -perhydroindolid, -2-methyl-perhydroindolid, -2,2-dimethyl-perhydroindolid, -1,2,3,4-tetrahydrochinolid, -2-methyl-1,2,3,4-tetrahydrochinolid, -perhydrochinolid, -2-methyl-perhydrochinolid, -1,2,3,4-tetrahydro-isochinolid und -perhydroisochinolid.

Die α-Hydroxy-carbonsäureamide der Formel (II) sind teilweise bekannt (vergleiche US-PS 3399988; DE-OS' 2201432 und 2647481). Sie können, wie im nachstehenden Schema skizziert, ausgehend von α-Chlorcarbonsäure-chloriden hergestellt werden:

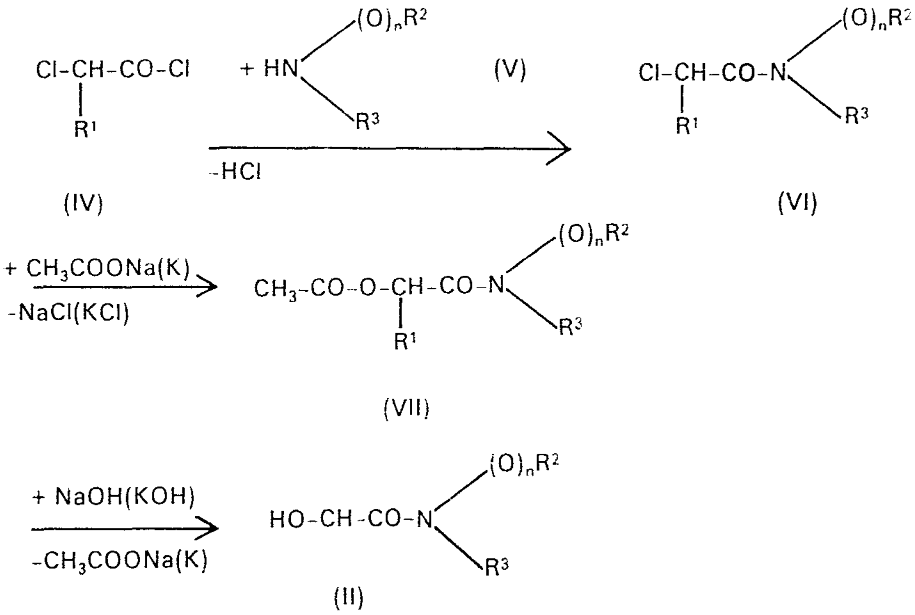

(IV) (V) (VI) (VII) (II)

Hierzu werden zunächst die literaturbekannten α-Chlorcarbonsäurechloride der Formel (IV) mit Aminen der Formel (V) - wobei n, $R^1$, $R^2$ und $R^3$ die oben angegebene Bedeutung haben - gegebenenfalls in Gegenwart eines Säurebindemittels wie z.B. Triethylamin und gegebenenfalls unter Verwendung eines inerten Verdünnungsmittels wie z.B. 1,2-Dichlorethan, bei Temperaturen zwischen -20 und 100°C, vorzugsweise zwischen -10 und 50°C in die entsprechenden Chlorcarbonsäureamide der Formel (VI) überführt. Die Aufarbeitung dieser Produkte erfolgt nach üblichen Methoden durch Waschen mit Wasser, Trocknen der organischen Phase und Abdestillieren des Lösungsmittels.

Die Verbindungen der Formel (VI) werden mit Natrium- oder Kalium-acetat, gegebenenfalls unter Verwendung eines Verdünnungsmittels wie z.B. Essigsäure oder Dimethylsulfoxid, bei Temperaturen zwischen 20 und 150°C, vorzugsweise zwischen 50 und 120°C, zu den entsprechenden α-Acetoxy-carbonsäureamiden der Formel (VII) umgesetzt. Soweit die Produkte hierbei kristallin anfallen, werden sie durch Absaugen isoliert. Andernfalls erfolgt die Aufarbeitung nach üblichen Methoden, beispielsweise durch Abdestillieren des Lösungsmittels im Vakuum, Aufnehmen des Rückstandes in Methylenchlorid, Waschen mit Wasser und Abdestillieren des Lösungsmittels.

Durch Umsetzung mit wässrig-alkoholischer Natronlauge oder Kalilauge bei Temperaturen

zwischen 0 und 100°C, vorzugsweise zwischen 10 und 50°C, können die Verbindungen der Formel (VII) zu den Verbindungen der Formel (II) entacyliert werden. Zur Isolierung der Produkte werden die Lösungsmittel im Vakuum abdestilliert, der Rückstand mit einem organischen Lösungsmittel, wie z.B. Methylenchlorid oder Essigester extrahiert, die Lösung getrocknet und das Lösungsmittel abdestilliert.

Als Ausgangsstoffe der Formel (III) seien beispielsweise die folgenden Halogentetrazole genannt:

5-Chlor- und 5-Brom-1-phenyl-(1H)-tetrazol,
5-Chlor- und 5-Brom-1-(4-chlor-2-methyl-phenyl)-(1H)-tetrazol,
5-Chlor- und 5-Brom-1-(2-chlor-6-methyl-phenyl)-(1H)-tetrazol,
5-Chlor- und 5-Brom-1-(3-nitro-phenyl)-(1H)-tetrazol,
5-Chlor- und 5-Brom-1-(4-methyl-phenyl)-(1H)-tetrazol,
5-Chlor- und 5-Brom-1-(2,5-dichlor-phenyl)-(1H)-tetrazol,
5-Chlor- und 5-Brom-1-(3,4-dichlor-phenyl)-(1H)-tetrazol,
5-Chlor- und 5-Brom-1-(3-trifluormethylphenyl)-(1H)-tetrazol,
5-Chlor- und 5-Brom-1-(2-Methyl-phenyl)-(1H)-tetrazol,
5-Chlor- und 5-Brom-1-(3-Methyl-phenyl)-(1H)-tetrazol,
5-Chlor- und 5-Brom-1-(3,4-dimethyl-phenyl)-(1H)-tetrazol,
5-Chlor- und 5-Brom-1-(2,4-dimethyl-phenyl)-(1H)-tetrazol.

Halogentetrazole der Formel (III) sind bereits bekannt (vgl. DE-AS 1 251 327 und GB-PS 1 128 025). Chlortetrazole der Formel (III) erhält man beispielsweise durch Umsetzung von Isocyanid-dichloriden der Formel

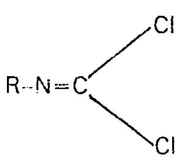 (VII)

in welcher
R die oben angegebene Bedeutung hat,
mit Natriumazid, gegebenenfalls in Gegenwart von Verdünnungsmitteln, wie z.B. Wasser und Aceton, bei Temperaturen zwischen 10 und 100°C.

Die kristallin anfallenden Produkte werden, gegebenenfalls nach Verdünnen mit Wasser, durch Vakuumfiltration isoliert.

Das Verfahren zur Herstellung der neuen Azolyloxycarbonsäureamide wird vorzugsweise unter Verwendung geeigneter Lösungs- oder Verdünnungsmittel durchgeführt. Als solche kommen praktisch alle organischen Solventien in Frage. Hierzu gehören insbesondere Alkohole, wie Methanol, Äthanol, n- und iso-Propanol, n-, iso-, sek.- und tert.-Butanol, Äther wie Diäthyläther, Dibutyläther, Tetrahydrofuran und Dioxan, Ketone wie Aceton, Methyläthylketon, Methylisopropylketon und Methylisobutylketon, Nitrile wie Acetonitril und Propionsäurenitril, sowie die hochpolaren Lösungsmittel Dimethylformamid, Dimethylsulfoxid, Sulfolan und Hexamethylphosphorsäuretriamid.

Als Säureakzeptoren können praktisch alle üblicherweise verwendbaren Säurebindemittel eingesetzt werden: hierzu gehören insbesondere Alkali- und Erdalkalihydroxide bzw. -oxide, z.B. Calcium-hydroxid, Alkali- und Erdalkali-carbonate wie Natrium-, Kalium- und Calciumcarbonat, Alkalialkoholate wie Natrium-methylat, -äthylat und -tert.-butylat, Kaliummethylat, -äthylat und -tert-butylat, ferner aliphatische, aromatische oder heterocyclische Amine wie Triäthylamin, Dimethylanilin, Dimethylbenzylamin, Pyridin, Diazobicyclooctan und Diazabicycloundecen.

Die Reaktionstemperatur kann innerhalb eines grösseren Bereichs variiert werden. Im allgemeinen arbeitet man zwischen −50 und +150°C, vorzugsweise bei −20 bis +100°C.

Das erfindungsgemässe Verfahren wird im allgemeinen bei Normaldruck durchgeführt.

Bei der Durchführung des erfindungsgemässen Verfahrens setzt man auf 1 Mol Halogenazol der Formel (III) 1,0 bis 1,5 Mol $\alpha$-Hydroxy-carbonsäureamid der Formel (II) ein. Die Umsetzung wird im allgemeinen in einem geeigneten Verdünnungsmittel durchgeführt und das Reaktionsgemisch wird bei der erforderlichen Temperatur mehrere Stunden gerührt.

Die Isolierung der Produkte erfolgt nach üblichen Methoden: Man destilliert gegebenenfalls einen Teil des Verdünnungsmittels unter vermindertem Druck ab und giesst den Rest der Reaktionsmischung in Wasser. Soweit die Produkte hierbei kristallin anfallen, werden sie durch Absaugen isoliert. Andernfalls werden die organischen Produkte mit einem mit Wasser nicht mischbaren Lösungsmittel wie z.B. Toluol oder Methylenchlorid extrahiert; nach Waschen und Trocknen wird dann von der organischen Phase das Lösungsmittel im Vakuum abdestilliert. Die zurückbleibenden Produkte werden durch ihren Schmelzpunkt bzw. ihren Brechungsindex charakterisiert.

Die erfindungsgemässen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab.

Die erfindungsgemässen Wirkstoffe können z.B. bei den folgenden Pflanzen verwendet werden:

Dikotyle Unkräuter der Gattungen: Sinapis,

Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemässen Wirkstoffe zeigen insbesondere neben einer sehr guten Wirkung gegen grasartige Unkräuter, einschliesslich Cyperus, auch eine gute herbizide Wirkung bei breitblättrigen Unkräutern. Ein selektiver Einsatz der erfindungsgemässen Wirkstoffe ist in verschiedenen Kulturen möglich, z.B. in Rüben, Sojabohnen, Baumwolle, Mais, Reis und anderen Getreidearten. Dabei sind einzelne Wirkstoffe als Selektivherbizide in Rüben, Baumwolle und Getreide besonders geeignet.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone wie Aceton, Methyläthylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silicate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnuss-Schalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykol-ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfrass, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen,

Emulsionen, Pulver, Pasten und Granulate angewandt werden. Die Anwendung geschieht in üblicher Weise, z.B. durch Giessen, Spritzen, Sprühen, Streuen oder Stäuben.

Die erfindungsgemässen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in grösseren Bereichen schwanken. Sie hängt im wesentlichen von der Art des gewünschten Effekts ab. Im allgemeinen liegen die Aufwandmengen zwischen 0,01 und 10 kg Wirkstoff pro ha, vorzugsweise zwischen 0,1 und 8 kg/ha.

Die erfindungsgemässen Wirkstoffe weisen zum Teil bei bestimmten Anwendungskonzentrationen auch eine wachstumsregulierende Wirkung auf.

Die nachfolgenden Beispiele dienen zur Erläuterung der Erfindung.

Herstellungsbeispiele:

Beispiel 1

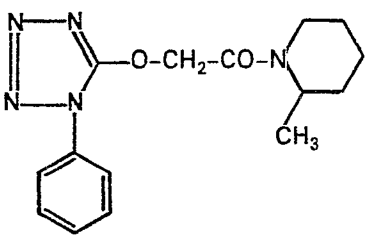

12,6 g (0,08 Mol) Hydroxyessigsäure-2-methylpiperidid und 14,4 g (0,08 Mol) 5-Chlor-1-phenyl-(1H)-tetrazol werden zu einer Lösung von 9,9 g (0,088 Mol) Kalium-tert.-butylat in 150 ml tert.-Butanol gegeben. Die Mischung wird ca. 15 Stunden bei 40°C gerührt und dann mit Toluol und Wasser verdünnt. Die organische Phase wird abgetrennt, mit Wasser gewaschen, mit Natriumsulfat getrocknet und filtriert. Vom Filtrat wird das Lösungsmittel unter vermindertem Druck sorgfältig abdestilliert. Man erhält als Rückstand 16,5 g (69% der Theorie) 1-Phenyl-5-tetrazolyloxyessigsäure-2-methyl-piperidid in Form eines hellbraunen, zähflüssigen Öls.

Elementaranalyse:

| | | | | |
|---|---|---|---|---|
| Ber.: | C 59,79% | H 6,35% | N 23,24% | O 10,6% |
| Gef.: | C 59,6 % | H 6,5 % | N 22,3 % | O 10,3% |

Analog Beispiel 1 können die nachstehend aufgeführten Verbindungen der Formel (I) hergestellt werden:

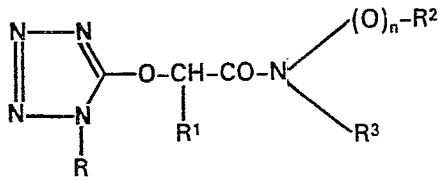 (I)

Tabelle

| Beispiel Nr. | R | $R^1$ | 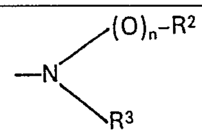 | Brechungsindex ($n_D^{20}$) oder Schmelzpunkt (°C) |
|---|---|---|---|---|
| 2 | $C_6H_5$ | H | 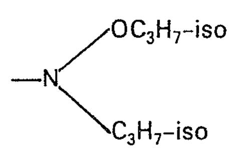 | 1,5672 |
| 3 | $C_6H_5$ | H | $-N(CH_2-CH=CH_2)_2$ | 79 |
| 4 | $C_6H_5$ | H | 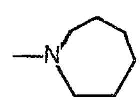 | 1,5618 |
| 5 | $C_6H_5$ | H | $-N(CH_3)_2$ | 105 |
| 6 | $C_6H_5$ | H | $-N(C_2H_5)_2$ | 50 |
| 7 | $C_6H_5$ | H | 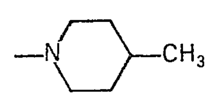 | 131 |

Tabelle (Fortsetzung)

| Beispiel Nr. | R | $R^1$ | 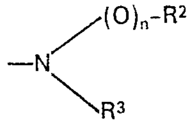 | Brechungsindex ($n_D^{20}$) oder Schmelzpunkt (°C) |
|---|---|---|---|---|
| 8 | $C_6H_5$ | H | 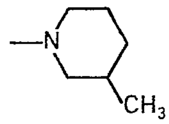 | 1,5572 |
| 9 | $C_6H_5$ | H | 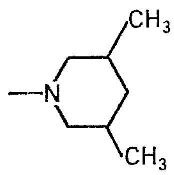 | 1,5464 |
| 10 | $C_6H_5$ | H | 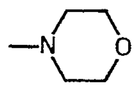 | 110 |
| 11 | $C_6H_5$ | H | $-N(CH_2CH_2CH_3)_2$ | 39 |
| 12 | $C_6H_5$ | H | 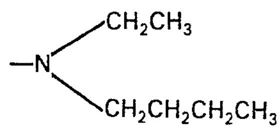 | 1,5360 |
| 13 | $C_6H_5$ | H | 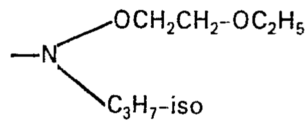 | 1,5116 |
| 14 | $C_6H_5$ | H | 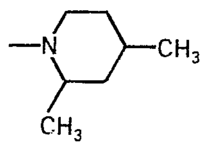 | 1,5469 |
| 15 | $C_6H_5$ | H | 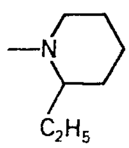 | 1,5482 |
| 16 | $C_6H_5$ | H | 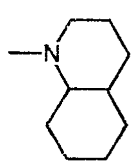 | 113 |
| 17 | $C_6H_5$ | H | 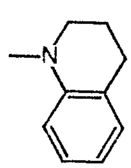 | 142 |

Tabelle (Fortsetzung)

| Beispiel Nr. | R | $R^1$ | 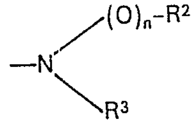 | Brechungsindex ($n_D^{20}$) oder Schmelzpunkt (°C) |
|---|---|---|---|---|
| 18 | $C_6H_5$ | H | 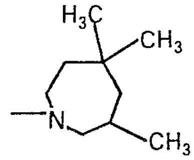 | 1,5394 |
| 19 | $C_6H_5$ | H | 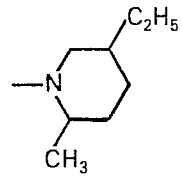 | 1,5389 |
| 20 | $C_6H_5$ | H | 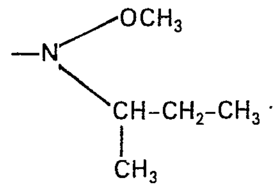 | 1,4783 |
| 21 | $C_6H_5$ | H | 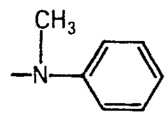 | 1,5397 |
| 22 | $C_6H_5$ | H | 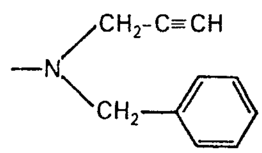 | 1,5770 |
| 23 | $C_6H_5$ | H | 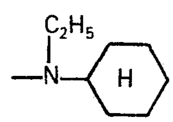 | 114 |
| 24 | $C_6H_5$ | H | $-N(CH_2CH_2OCH_3)_2$ | 1,5312 |
| 25 | 2,5-$Cl_2$-$C_6H_3$ | H | 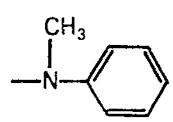 | 1,5619 |
| 26 | 3,4-$Cl_2$-$C_6H_3$ | H | 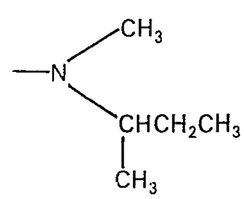 | 1,5723 |
| 27 | 3,4-$Cl_2$-$C_6H_3$ | H | 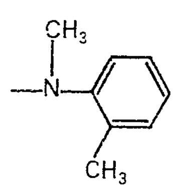 | 45–8°C |

Tabelle (Fortsetzung)

| Beispiel Nr. | R | $R^1$ | 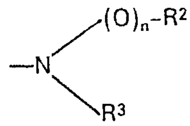 | Brechungsindex ($n_D^{20}$) oder Schmelzpunkt (°C) |
|---|---|---|---|---|
| 28 | $3,4\text{-}Cl_2\text{-}C_6H_3$ | H | 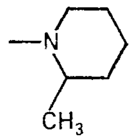 | 1,5801 |
| 29 | $C_6H_5$ | H | 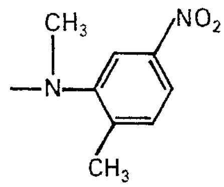 | 137 |
| 30 | $C_6H_5$ | H | 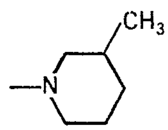 | 1,5477 |
| 31 | $C_6H_5$ | H | 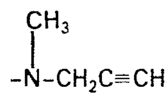 | 1,5298 |
| 32 | $3,5\text{-}(CF_3)_2\text{-}C_6H_3$ | H | 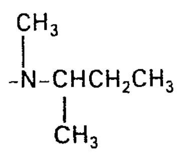 | 1,4851 |
| 33 | $3,5\text{-}(CF_3)_2\text{-}C_6H_3$ | H | 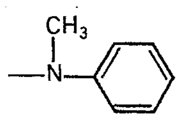 | 1,4992 |
| 34 | $3,5\text{-}(CF_3)_2\text{-}C_6H_3$ | H | $-N(CH_2CH_2OCH_3)_2$ | 82°C |
| 35 | $3,4\text{-}Cl_2\text{-}C_6H_3$ | H | 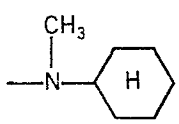 | 1,5480 |
| 36 | $C_6H_5$ | H | 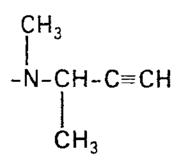 | 112°C |
| 37 | $2,5\text{-}Cl_2\text{-}C_6H_3$ | H | 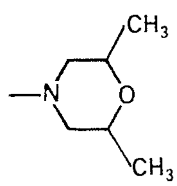 | 1,5614 |

Tabelle (Fortsetzung)

| Beispiel Nr. | R | $R^1$ | 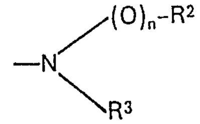 | Brechungsindex ($n_D^{20}$) oder Schmelzpunkt (°C) |
|---|---|---|---|---|
| 38 | 2,5-$Cl_2$-$C_6H_3$ | H | 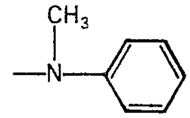 | 153°C |
| 39 | 2,5-$Cl_2$-$C_6H_3$ | H | 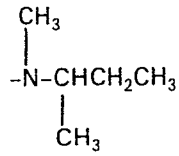 | 1,4991 |
| 40 | 2,5-$Cl_2$-$C_6H_3$ | H | 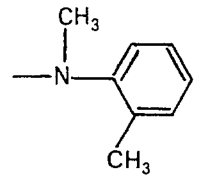 | 134°C |
| 41 | 3,5-$(CF_3)_2$-$C_6H_3$ | H | 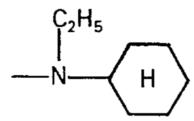 | 112°C |
| 42 | 3,5-$(CF_3)_2$-$C_6H_3$ | H | 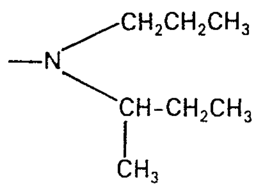 | 1,5238 |
| 43 | 3,5-$(CF_3)_2$-$C_6H_3$ | H | 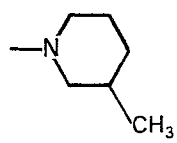 | 1,5015 |
| 44 | 3,5-$(CF_3)_2$-$C_6H_3$ | H | 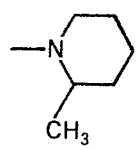 | 1,5193 |
| 45 | 3,5-$(CF_3)_2$-$C_6H_3$ | H | 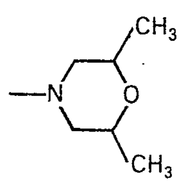 | 1,5316 |
| 46 | 3,5-$(CF_3)_2$-$C_6H_3$ | H | 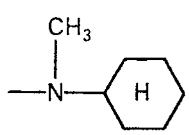 | 1,5419 |
| 47 | 2,5-$Cl_2$-$C_6H_3$ | H | $-N(CH_2CH_2-OCH_3)_2$ | 1,5298 |

Tabelle (Fortsetzung)

| Beispiel Nr. | R | $R^1$ | 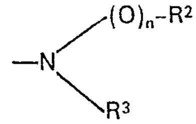 | Brechungsindex ($n_D^{20}$) oder Schmelzpunkt (°C) |
|---|---|---|---|---|
| 48 | 2,5-$Cl_2$-$C_6H_3$ | H | 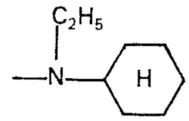 | 1,5085 |
| 49 | 2,5-$Cl_2$-$C_6H_3$ | H | 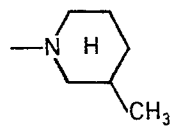 | 130-3°C |
| 50 | 3-$CF_3$-$C_6H_4$ | H | 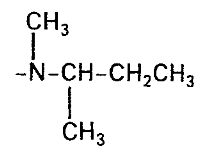 | 1,4993 |
| 51 | 3-$CF_3$-$C_6H_4$ | H | 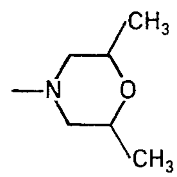 | 1,5207 |
| 52 | 3-$CF_3$-$C_6H_4$ | H | 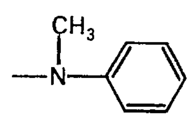 | 1,5082 |
| 53 | 3-$NO_2$-$C_6H_4$ | H | 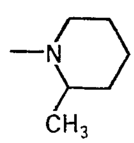 | 124°C |
| 54 | 3-$NO_2$-$C_6H_4$ | H | 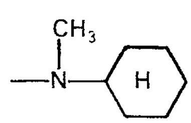 | 1,5411 |
| 55 | 3-$NO_2$-$C_6H_4$ | H | 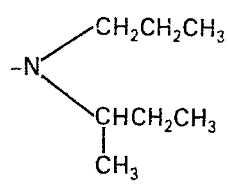 | 1,5332 |
| 56 | 3-$CF_3$-$C_6H_4$ | H | 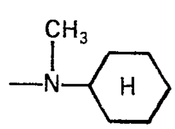 | 1,5335 |
| 57 | 3-$CF_3$-$C_6H_4$ | H | 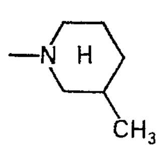 | 96°C |

Tabelle (Fortsetzung)

| Beispiel Nr. | R | $R^1$ | 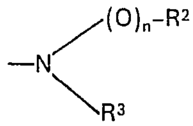 | Brechungsindex ($n_D^{20}$) oder Schmelzpunkt (°C) |
|---|---|---|---|---|
| 58 | $3-CF_3-C_6H_4$ | H | 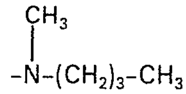 | 1,4985 |
| 59 | $3-CF_3-C_6H_4$ | H | 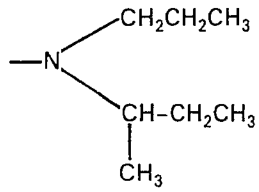 | 1,4960 |
| 60 | $2,5-Cl_2-C_6H_3$ | H | 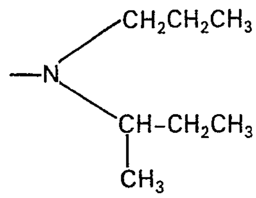 | 1,5500 |
| 61 | $2,5-Cl_2-C_6H_3$ | H | 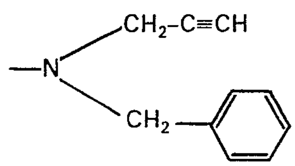 | 1,4979 |
| 62 | $3-CF_3-C_6H_4$ | H | 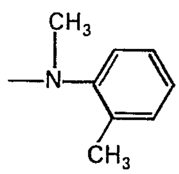 | 1,5162 |
| 63 | $3-CF_3-C_6H_4$ | H | 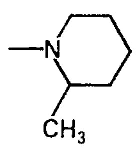 | 1,5180 |
| 64 | $3-CF_3-C_6H_4$ | H | 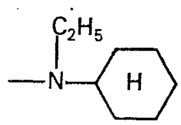 | 1,5135 |
| 65 | $3-CF_3-C_6H_4$ | H | $-N(CH_2CH_2OCH_3)_2$ | 1,4958 |
| 66 | $2,5-Cl_2-C_6H_3$ | H | 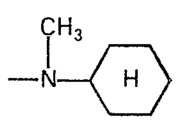 | 110°C |
| 67 | $2,5-Cl_2-C_6H_3$ | H | 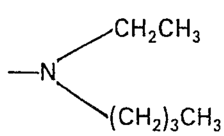 | 1,5436 |

Tabelle (Fortsetzung)

| Beispiel Nr. | R | $R^1$ | 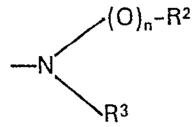 | Brechungsindex ($n_D^{20}$) oder Schmelzpunkt (°C) |
|---|---|---|---|---|
| 68 | $2,5\text{-}Cl_2\text{-}C_6H_3$ | H | 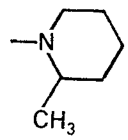 | 1,5509 |
| 69 | $3\text{-}NO_2\text{-}C_6H_4$ | H | 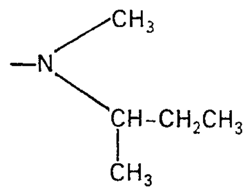 | 1,5472 |
| 70 | $3\text{-}NO_2\text{-}C_6H_4$ | H | 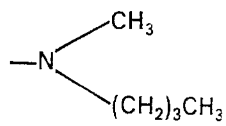 | 1,5398 |
| 71 | $3\text{-}NO_2\text{-}C_6H_4$ | H | 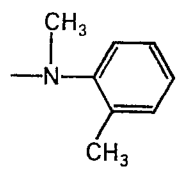 | 140°C |
| 72 | $3\text{-}NO_2\text{-}C_6H_4$ | H | 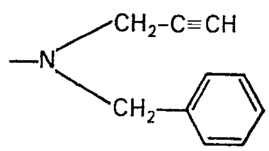 | 1,5462 |
| 73 | $3\text{-}NO_2\text{-}C_6H_4$ | H | $-N(CH_2CH_2OCH_3)_2$ | 1,5437 |
| 74 | $3\text{-}NO_2\text{-}C_6H_4$ | H | 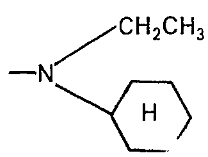 | 1,5388 |
| 75 | $3\text{-}NO_2\text{-}C_6H_4$ | H | 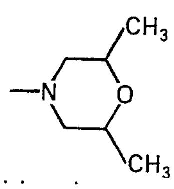 | 83°C |
| 76 | $4\text{-}CH_3\text{-}C_6H_4$ | H | 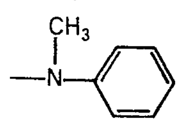 | 99°C |
| 77 | $4\text{-}CH_3\text{-}C_6H_4$ | H | 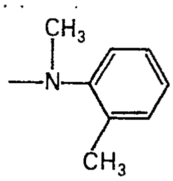 | 129–132°C |

Tabelle (Fortsetzung)

| Beispiel Nr. | R | $R^1$ | 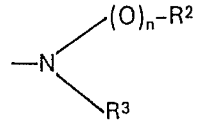 | Brechungsindex ($n_D^{20}$) oder Schmelzpunkt (°C) |
|---|---|---|---|---|
| 78 | $4-CH_3-C_6H_4$ | H | $-N(CH_2CH_2OCH_3)_2$ | 95°C |
| 79 | $2-Cl-6-CH_3-C_6H_3$ | H | 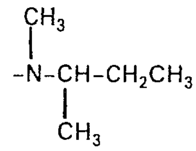 | 1,5350 |
| 80 | $2-Cl-6-CH_3-C_6H_3$ | H | 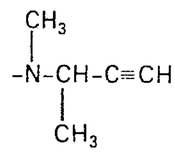 | 130°C |
| 81 | $2-Cl-6-CH_3-C_6H_3$ | H | 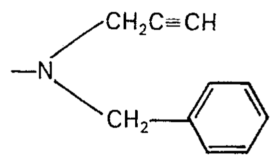 | 137°C |
| 82 | $2-Cl-6-CH_3-C_6H_3$ | H | $-N(CH_2CH_2OCH_3)_2$ | 1,5303 |
| 83 | $2-Cl-6-CH_3-C_6H_3$ | H | 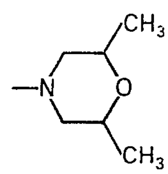 | 1,5469 |
| 84 | $2-Cl-6-CH_3-C_6H_3$ | H | 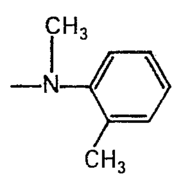 | 144°C |
| 85 | $2-Cl-6-CH_3-C_6H_3$ | H | 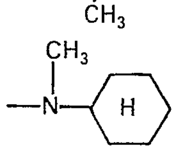 | 173°C |
| 86 | $2-Cl-6-CH_3-C_6H_3$ | H | 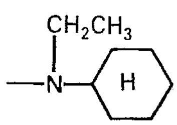 | 44°C |
| 87 | $4-Cl-2-CH_3-C_6H_3$ | H | 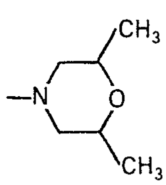 | 65°C |
| 88 | $4-Cl-2-CH_3-C_6H_3$ | H | 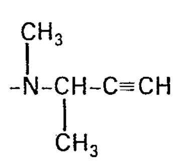 | 116°C |

Tabelle (Fortsetzung)

| Beispiel Nr. | R | $R^1$ | 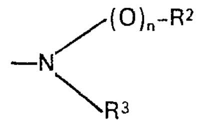 | Brechungsindex ($n_D^{20}$) oder Schmelzpunkt (°C) |
|---|---|---|---|---|
| 89 | $4\text{-}Cl\text{-}2\text{-}CH_3\text{-}C_6H_3$ | H | 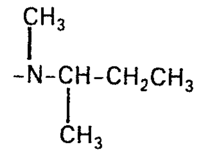 | 1,5478 |
| 90 | $4\text{-}Cl\text{-}2\text{-}CH_3\text{-}C_6H_3$ | H | $-N(CH_2CH_2OCH_3)_2$ | 1,5431 |
| 91 | $4\text{-}Cl\text{-}2\text{-}CH_3\text{-}C_6H_3$ | H | 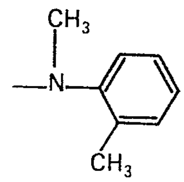 | 152°C |
| 92 | $4\text{-}Cl\text{-}2\text{-}CH_3\text{-}C_6H_3$ | H | 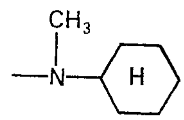 | 1,5423 |
| 93 | $4\text{-}Cl\text{-}2\text{-}CH_3\text{-}C_6H_3$ | H | 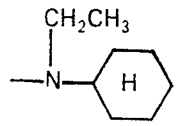 | 1,5395 |
| 94 | $4\text{-}Cl\text{-}2\text{-}CH_3\text{-}C_6H_3$ | H | 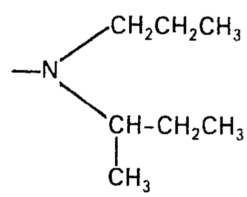 | 1,5331 |
| 95 | $4\text{-}Cl\text{-}2\text{-}CH_3\text{-}C_6H_3$ | H | 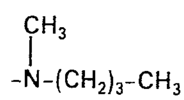 | 1,5430 |
| 96 | $4\text{-}Cl\text{-}2\text{-}CH_3\text{-}C_6H_3$ | H | 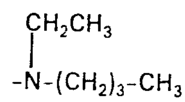 | 1,5382 |
| 97 | $C_6H_5$ | H | 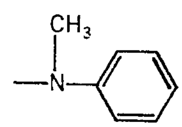 | 153°C |
| 98 | $C_6H_5$ | H | 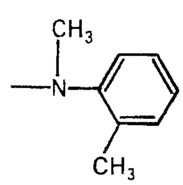 | 139-141°C |
| 99 | $C_6H_5$ | H | 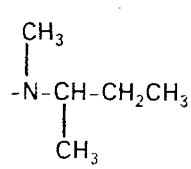 | 1,5291 |

Tabelle (Fortsetzung)

| Beispiel Nr. | R | $R^1$ | 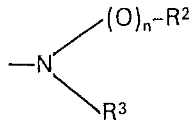 | Brechungsindex ($n_D^{20}$) oder Schmelzpunkt (°C) |
|---|---|---|---|---|
| 100 | $C_6H_5$ | H | 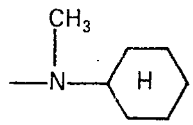 | 116°C |
| 101 | $C_6H_5$ | H | 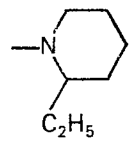 | 1,5237 |
| 102 | $C_6H_5$ | H | 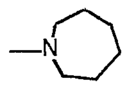 | 1,5409 |
| 103 | $C_6H_5$ | H | 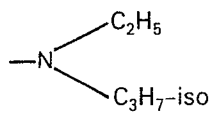 | 1,5385 |
| 104 | $C_6H_5$ | H | 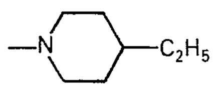 | 1,5465 |
| 105 | 3,5-$(CF_3)_2C_6H_3$ | H | 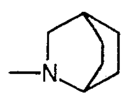 | 89 |
| 106 | 2,4-$Cl_2$-$C_6H_3$ | H | 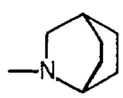 | 142 |
| 107 | 3-$CF_3$-$C_6H_4$ | H | 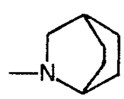 | 69 |
| 108 | $C_6H_5$ | H | 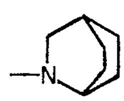 | 110 |
| 109 | 3-$NO_2$-$C_6H_4$ | H | 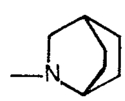 | 152 |
| 110 | 2,5-$Cl_2$-$C_6H_3$ | H | 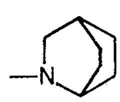 | 123 |
| 111 | 2,4-$Cl_2$-$C_6H_3$ | H | 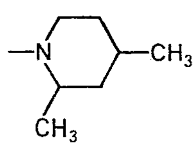 | 1,5543 |

Tabelle (Fortsetzung)

| Beispiel Nr. | R | $R^1$ | 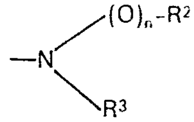 | Brechungsindex ($n_D^{20}$) oder Schmelzpunkt (°C) |
|---|---|---|---|---|
| 112 | 2,4-$Cl_2$-$C_6H_3$ | H | 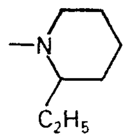 | 1,5575 |
| 113 | 2,4-$Cl_2$-$C_6H_3$ | H | 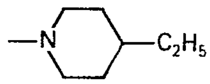 | 1,5542 |
| 114 | 2,4-$Cl_2$-$C_6H_3$ | H | 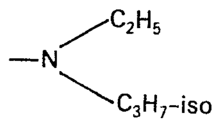 | 1,5433 |
| 115 | 3-$CF_3$-$C_6H_4$ | H | 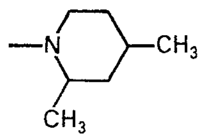 | 1,5054 |
| 116 | 3-$CF_3$-$C_6H_4$ | H | 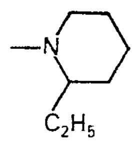 | 1,5029 |
| 117 | 3-$CF_3$-$C_6H_4$ | H | 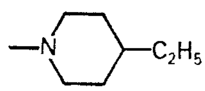 | 1,5065 |
| 118 | 3-$CF_3$-$C_6H_4$ | H | 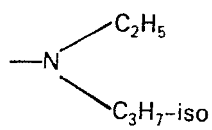 | 1,4923 |
| 119 | 2-$CH_3$-4-Cl-$C_6H_3$ | H | 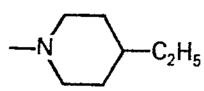 | 120 |
| 120 | 2-$CH_3$-4-Cl-$C_6H_3$ | H | 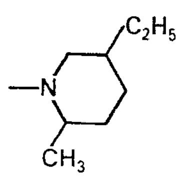 | 1,5397 |
| 121 | 2-$CH_3$-4-Cl-$C_6H_3$ | H | 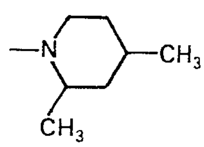 | 1,5389 |
| 122 | 2-$CH_3$-4-Cl-$C_6H_3$ | H | 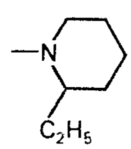 | 1,5475 |

Tabelle (Fortsetzung)

| Beispiel Nr. | R | $R^1$ | 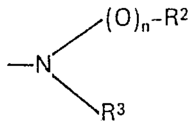 | Brechungsindex ($n_D^{20}$) oder Schmelzpunkt (°C) |
|---|---|---|---|---|
| 123 | 2-$CH_3$-4-Cl-$C_6H_3$ | H | 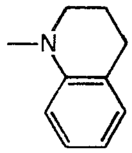 | 111 |
| 124 | 2-$CH_3$-4-Cl-$C_6H_3$ | H | 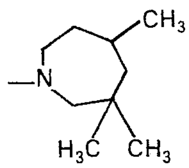 | 141 |
| 125 | $C_6H_5$ | H | 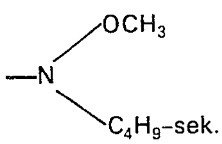 | (Oil) |
| 126 | 2,5-$(CF_3)_2$-$C_6H_3$ | H | 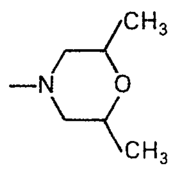 | (Oil) |
| 127 | 2-Cl-6-$CH_3$-$C_6H_3$ | H | 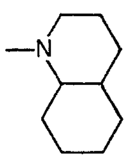 | 161 |
| 128 | 2-Cl-6-$CH_3$-$C_6H_3$ | H | 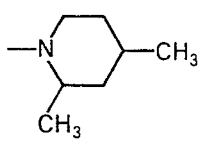 | 146 |
| 129 | 2-Cl-6-$CH_3$-$C_6H_3$ | H | 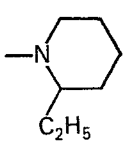 | 126 |
| 130 | 2-Cl-6-$CH_3$-$C_6H_3$ | H | 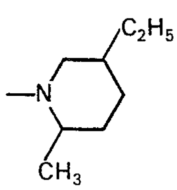 | 1,5372 |
| 131 | 2-Cl-6-$CH_3$-$C_6H_3$ | H | 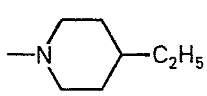 | 86 |

Die als Ausgangsstoffe zu verwendenden Verbindungen der Formel (II) können wie folgt hergestellt werden:

Beispiel a:

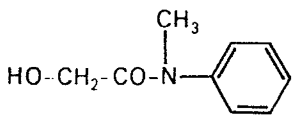

Eine Suspension aus 183,5 g (1 Mol) Chloressigsäure-N-methylanilid, 82 g (1 Mol) wasserfreiem Natriumacetat und 320 ml Toluol wird 4 Stunden auf 115-120°C erhitzt und dann auf Raumtemperatur abgekühlt. Der Ansatz wird abgesaugt und der Rückstand mit kaltem Toluol nachgewaschen. Aus der toluolischen Lösung werden nach Abdestillieren des Lösungsmittels und Eindampfen des Rückstandes im Dampfstrahlvakuum bei einer Badtemperatur von 80-85°C 207 g α-Acetoxy-essigsäure-N-methylanilid erhalten, das beim Stehen kristallisiert. GC: = 98%ig, Schmelzpunkt 54-56°C; Ausbeute 99% der Theorie.

Das Reaktionsgemisch aus 211,2 g (1 Mol) α-Acetoxyessigsäure-N-methylanilid (98%ig), 0,2 g Natriumhydroxid und 160 g Methanol wird 4 Stunden unter Rückfluss erhitzt. Das Gemisch aus Methanol und Methylacetat wird abdestilliert. Der flüssige Destillationsrückstand - 170 g Ausbeute an Hydroxy-essigsäure-N-methylanilid, quantitativ, GC: 98%, Schmelzpunkt: 52-53°C, erstarrt beim Abkühlen.

Analog erhält man die nachstehenden Verbindungen der Formel (II):

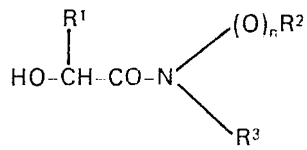

| Beispiel | $R^1$ | 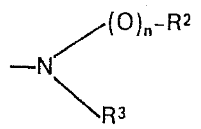 | Schmelzpunkt (°C); Brechungsindex; |
|---|---|---|---|
| b | H | 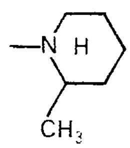 | 36 |
| c | H | $-N(CH_2-CH_2-OCH_3)_2$ | $n_D^{25}$: 1,4662 |
| d | H | 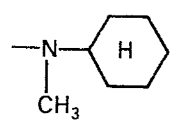 | 83 |
| e | H | 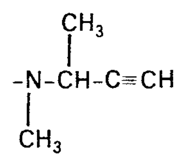 | $n_D^{25}$: 1,4859 |
| f | H | 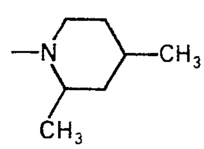 | $n_D^{23}$: 1,4816 |
| g | H | 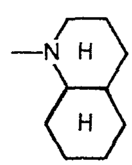 | 55 |

Tabelle (Fortsetzung)

| Beispiel | $R^1$ | 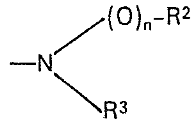 | Siedepunkt/Druck oder Schmelzpunkt (3°C); Brechungsindex; |
|---|---|---|---|
| h | H | 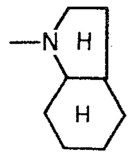 | $n_D^{23}$: 1,5076 |
| i | H | 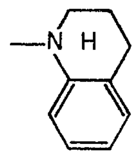 | 80 |
| j | H | 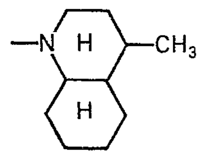 | |
| k | H | 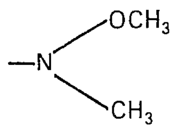 | $n_D^{22}$: 1,4583<br>Sdp. 47°C/0,133 mbar |
| l | H | 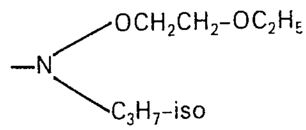 | $n_D^{24}$: 1,4485 |
| m | H | 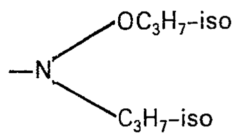 | $n_D^{21}$: 1,4475 |
| n | H | 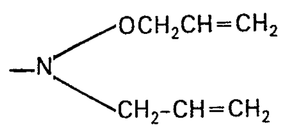 | $n_D^{23}$: 1,4793<br>Sdp. 76–78°C/0,0133 mbar |

Die als Ausgangsstoffe zu verwendenden Halogentetrazole der Formel (III) können beispielsweise wie folgt hergestellt werden:

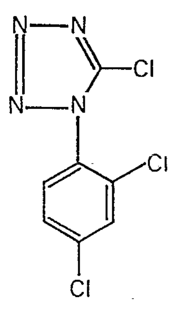

Eine Lösung von 243 g (1,0 Mol) 2,4-Dichlorphenyl-isocyanid-dichlorid in 800 ml Aceton wird tropfenweise zu einer Lösung von 65 g (1,0 Mol) Natriumazid in 1 l Wasser gegeben. Das Reaktionsgemisch wird 30 Minuten bei 50°C und weitere 30 Minuten unter Rückfluss gerührt, abgekühlt, in Wasser gegossen und abgesaugt. Man erhält 243 g (97% der Theorie) 5-Chlor-1-(2,4-dichlorphenyl-(1H)-tetrazol vom Schmelzpunkt 81°C.

Analog erhält man

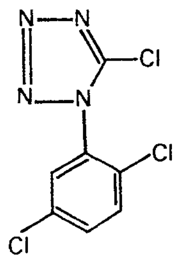

Schmelzpunkt 122°C

Beispiel A
Pre-emergence-Test

Lösungsmittel: 5 Gewichtsteile Aceton
Emulgator: 1 Gewichtsteil Alkylarylpolyglycoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Bei diesem Test zeigen z.B. die folgenden Verbindungen der Herstellungsbeispiele eine ausgezeichnete Wirkung: Nr. 1, 3, 4, 8, 14, 15, 16, 21, 28, 30, 31, 36, 97, 98, 99, 100, 101, 102.

**Patentansprüche**

1. Tetrazolyloxycarbonsäureamide der Formel

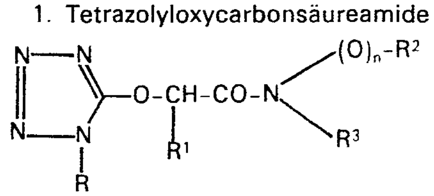

(I)

in welcher

R für Phenyl steht, welches gegebenenfalls substituiert ist durch Chlor, Nitro, Methyl und/oder Trifluormethyl, wobei auch mehrfache und gemischte Substitutionen durch die genannten Reste möglich ist, in welcher weiter

$R^1$ für Wasserstoff steht,

n für Null oder 1 steht,

$R^2$ für $C_1$-$C_6$-Alkyl, $C_1$-$C_4$-Alkoxy-ethyl, Allyl, Propargyl, 1-Methyl-propargyl oder 1,1-Dimethyl-propargyl oder - für den Fall, dass n für Null steht - auch für Cyanomethyl, Cyclopentyl, Cyclohexyl, Benzyl oder Phenyl steht,

in welcher weiter

$R^3$ für $C_1$-$C_6$-Alkyl, $C_1$-$C_4$-Alkoxy-ethyl, Allyl, Propargyl, 1-Methyl-propargyl, 1,1-Dimethyl-propargyl, Cyanomethyl, Cyclopentyl, Cyclohexyl, Benzyl, Naphtyl oder Phenyl steht, welches gegebenenfalls durch Methyl, Chlor, Cyano, Nitro oder Methoxy substituiert ist, wobei auch mehrfache und gemischte Substitution durch die genannten Reste möglich ist,

in welcher weiter - für den Fall, dass n für Null steht - die Reste $R^2$ und $R^3$ zusammen mit dem Stickstoffatom, an das sie gebunden sind, für Pyrrolidyl, Monoalkyl- oder Dialkyl-pyrrolidyl mit 1 bis 3 Kohlenstoffatomen je Alkylgruppe, Morpholinyl oder Dialkylmorpholinyl mit 1 bis 3 Kohlenstoffatomen je Alkylgruppe, Piperidyl, Monoalkyl-, Dialkyl-, oder Trialkylpiperidyl mit 1 bis 3 Kohlenstoffatomen je Alkylgruppe, für 4,4-Dialkoxy-piperidyl mit 1 bis 3 Kohlenstoffatomen je Alkoxygruppe, für spirosubstituiertes Piperidyl der Formel

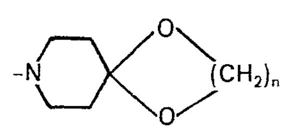

(worin n für 2 oder 3 steht), für Perhydroazepinyl (Hexamethylenimino-Rest), Trimethyl-perhydroazepinyl, für den Heptamethylenimino-Rest, für den Dodekamethylenimino-Rest, für 1,2,3,4-Tetrahydroindolyl, für Monoalkyl-, Dialkyl- oder Trialkyl-1,2,3,4-tetrahydroindolyl mit bis zu 3 Kohlenstoffatomen je Alkylgruppe, für Perhydroindolyl, Monoalkyl-, Dialkyl- oder Trialkyl-perhydroindolyl mit 1 bis 3 Kohlenstoffatomen je Alkylgruppe, 1,2,3,4-Tetrahydrochinolyl oder 1,2,3,4-Tetrahydro-iso-chinolyl, Monoalkyl-, Dialkyl- oder Trialkyl-1,2,3,4-tetrahydrochinolyl oder -isochinolyl mit 1 bis 3 Kohlenstoffatomen je Alkylgruppe, für Perhydrochinolyl oder Perhydro-isochinolyl, Monoalkyl-, -Dialkyl- oder Trialkylperhydrochinolyl oder -perhydroisochinolyl mit 1 bis 3 Kohlenstoffatomen je Alkylgruppe, für Perhydrothiazolyl, für Perhydrooxazolyl, für Perhydrooxazinyl, für den Rest

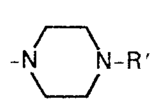

(worin R′ für $C_1$-$C_4$-Alkyl, für gegebenenfalls durch $C_1$-$C_2$-Alkyl, $C_1$-$C_2$-Alkoxy, Fluor, Chlor, Brom, Trifluormethyl und/oder Nitro substituiertes Phenyl, für Benzyl oder Phenyläthyl steht) oder für den Rest

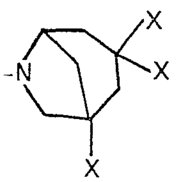

(worin X Wasserstoff oder Methyl bedeutet) stehen.

2. Verfahren zur Herstellung von Tetrazolyloxycarbonsäureamiden der Formel

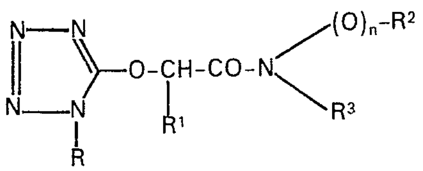

(I)

in welcher

R für Phenyl steht, welches gegebenenfalls substituiert ist duch Chlor, Nitro, Methyl und/oder Trifluormethyl, wobei auch mehrfache und gemischte Substitutionen durch die genannten Reste möglich ist, in welcher weiter

$R^1$ für Wasserstoff steht,

n für Null oder 1 steht,

$R^2$ für $C_1$-$C_6$-Alkyl, $C_1$-$C_4$-Alkoxy-ethyl, Allyl, Propargyl, 1-Methyl-propargyl oder 1,1-Dimethylpropargyl oder – für den Fall, dass n für Null steht – auch für Cyanomethyl, Cyclopentyl, Cyclohexyl, Benzyl oder Phenyl steht,

in welcher weiter

$R^3$ für $C_1$-$C_6$-Alkyl, $C_1$-$C_4$-Alkoxy-ethyl, Allyl, Propargyl, 1-Methyl-propargyl, 1,1-Dimethyl-propargyl, Cyanomethyl, Cyclopentyl, Cyclohexyl, Benzyl, Naphtyl oder Phenyl steht, welches gegebenenfalls durch Methyl, Chlor, Cyano, Nitro oder Methoxy substituiert ist, wobei auch mehrfache und gemischte Substitution durch die genannten Reste möglich ist,

in welcher weiter – für den Fall, dass n für Null steht – die Reste $R^2$ und $R^3$ zusammen mit dem Stickstoffatom, an das sie gebunden sind, für Pyrrolidyl, Monoalkyl- oder Dialkyl-pyrrolidyl mit 1 bis 3 Kohlenstoffatomen je Alkylgruppe, Morpholinyl oder Dialkylmorpholinyl mit 1 bis 3 Kohlenstoffatomen je Alkylgruppe, Piperidyl, Monoalkyl-, Dialkyl-, oder Trialkylpiperidyl mit 1 bis 3 Kohlenstoffatomen je Alkylgruppe, für 4,4-Dialkoxy-piperidyl mit 1 bis 3 Kohlenstoffatomen je Alkoxygruppe, für spirosubstituiertes Piperidyl der Formel

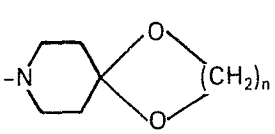

(worin n für 2 oder 3 steht), für Perhydroazepinyl (Hexamethylenimino-Rest), Trimethyl-perhydroazepinyl, für den Heptamethylenimino-Rest, für den Dodekamethylenimino-Rest, für 1,2,3,4-Tetrahydroindolyl, für Monoalkyl-, Dialkyl- oder Trialkyl-1,2,3,4-tetrahydroindolyl mit bis zu 3 Kohlenstoffatomen je Alkylgruppe, für Perhydroindolyl, Monoalkyl-, Dialkyl- oder Trialkyl-perhydroindolyl mit 1 bis 3 Kohlenstoffatomen je Alkylgruppe, 1,2,3,4-Tetrahydrochinolyl oder 1,2,3,4-Tetrahydro-iso-chinolyl, Monoalkyl-, Dialkyl- oder Trialkyl-1,2,3,4-tetrahydrochinolyl oder -isochinolyl mit 1 bis 3 Kohlenstoffatomen je Alkylgruppe, für Perhydrochinolyl oder Perhydro-isochinolyl, Monoalkyl-, -Dialkyl- oder Trialkylperhydrochinolyl oder -perhydroisochinolyl mit 1 bis 3 Kohlenstoffatomen je Alkylgruppe, für Perhydrothiazolyl, für Perhydrooxazolyl, für Perhydrooxazinyl, für den Rest

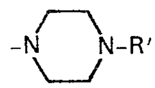

(worin R′ für $C_1$-$C_4$-Alkyl, für gegebenenfalls durch $C_1$-$C_2$-Alkyl, $C_1$-$C_2$-Alkoxy, Fluor, Chlor, Brom, Trifluormethyl und/oder Nitro substituiertes Phenyl, für Benzyl oder Phenyläthyl steht) oder für den Rest

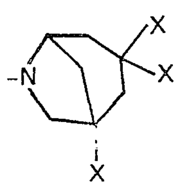

(worin X Wasserstoff oder Methyl bedeutet) stehen,

dadurch gekennzeichnet, dass man α-Hydroxycarbonsäureamide der Formel

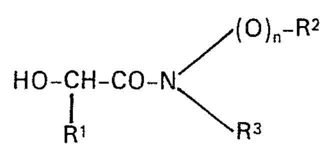

(II)

in welcher

n, $R^1$, $R^2$ und $R^3$ die oben angegebene Bedeutung haben, mit Halogentetrazolen der Formel

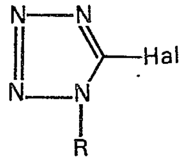

(III)

in welcher

R die oben angegebene Bedeutung hat und Hal für Chlor, Brom oder Jod steht,

gegebenenfalls in Gegenwart eines Säureakzeptors und gegebenenfalls unter Verwendung eines Verdünnungsmittels umsetzt.

3. Herbizide Mittel, gekennzeichnet durch einen Gehalt an Tetrazolyloxycarbonsäureamiden gemäss Anspruch 1.

4. Verwendung von Tetrazolyloxycarbonsäureamiden gemäss Anspruch 1 zur Bekämpfung von Pflanzenwachstum.

5. Verfahren zur Herstellung von herbiziden Mitteln, dadurch gekennzeichnet, dass man Tetrazolyloxycarbonsäureamide gemäss Anspruch 1 mit Streckmitteln und/oder oberflächenaktiven Mitteln vermischt.

**Claims**

1. Tetrazolyloxycarboxylic acid amides of the formula

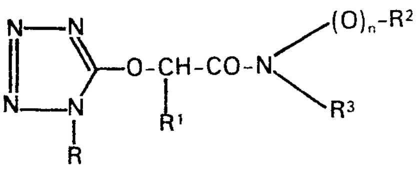

(I)

in which
R represents phenyl which is optionally substituted by chlorine, nitro, methyl and/or trifluoromethyl, multiple and mixed substitutions by the indicated radicals also being possible,
in which furthermore
$R^1$ represents hydrogen,
n represents zero or 1,
$R^2$ represents $C_1$-$C_6$-alkyl, $C_1$-$C_4$-alkoxy-ethyl, allyl, propargyl, 1-methyl-propargyl or 1,1-dimethyl-propargyl, or - in the case where n represents zero - also cyanomethyl, cyclopentyl, cyclohexyl, benzyl or phenyl,
in which furthermore
$R^3$ represents $C_1$-$C_6$-alkyl, $C_1$-$C_4$-alkoxy-ethyl, allyl, propargyl, 1-methyl-propargyl, 1,1-dimethyl-propargyl, cyanomethyl, cyclopentyl, cyclohexyl, benzyl, naphthyl or phenyl, which is optionally substituted by methyl, chlorine, cyano, nitro or methoxy, multiple and mixed substitution by the indicated radicals also being possible
in which furthermore - in the case where n represents zero - the radicals $R^2$ and $R^3$, together with the nitrogen atom to which they are bonded, represent pyrrolidyl, monoalkyl- or dialkyl-pyrrolidyl with 1 to 3 carbon atoms per alkyl group, morpholinyl or dialkylmorpholinyl with 1 to 3 carbon atoms per alkyl group, piperidyl, monoalkyl-, dialkyl- or trialkyl-piperidyl with 1 to 3 carbon atoms per alkyl group, 4,4-dialkoxy-piperidyl with 1 to 3 carbon atoms per alkoxy group, spiro-substituted piperidyl of the formula

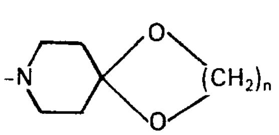

(wherein
n represents 2 or 3),
perhydroazepinyl (hexamethyleneimino radical), trimethyl-perhydroazepinyl, the heptamethyleneimino radical, the dodecamethyleneimino radical, 1,2,3,4-tetrahydroindolyl, monoalkyl-, dialkyl- or trialkyl-1,2,3,4-tetrahydroindolyl with up to 3 carbon atoms per alkyl group, perhydroindolyl, monoalkyl-, dialkyl- or trialkyl-perhydroindolyl with 1 to 3 carbon atoms per alkyl group, 1,2,3,4-tetrahydroquinolyl or 1,2,3,4-tetrahydro-iso-quinolyl, monoalkyl-, dialkyl- or trialkyl-1,2,3,4-tetrahydro-quinolyl or -isoquinolyl with 1 to 3 carbon atoms per alkyl group, perhydro-quinolyl or perhydro-iso-quinolyl, monoalkyl-, dialkyl- or trialkyl-perhydroquinolyl or -perhydroisoquinolyl with 1 to 3 carbon atoms per alkyl group, perhydrothiazolyl, perhydrooxazolyl, perhydrooxazinyl, the radical

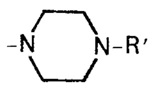

(wherein
R' represents $C_1$-$C_4$-alkyl, phenyl which is optionally substituted by $C_1$-$C_2$-alkyl, $C_1$-$C_2$-alkoxy, fluorine, chlorine, bromine, trifluoromethyl and/or nitro, or benzyl or phenylethyl) or represents the radical

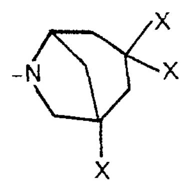

(wherein
X represents hydrogen or methyl).

2. Process for the preparation of tetrazolyloxycarboxylic acid amides of the formula

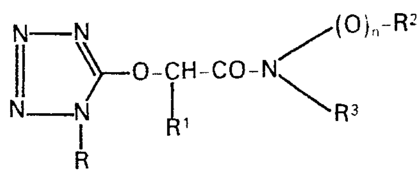

(I)

in which
R represents phenyl which is optionally substituted by chlorine, nitro, methyl and/or trifluoromethyl, multiple and mixed substitutions by the indicated radicals also being possible,
in which furthermore
$R^1$ represents hydrogen,
n represents zero or 1,
$R^2$ represents $C_1$-$C_6$-alkyl, $C_1$-$C_4$-alkoxy-ethyl, allyl, propargyl, 1-methyl-propargyl or 1,1-dimethyl-propargyl, or - in the case where n represents zero - also cyanomethyl, cyclopentyl, cyclohexyl, benzyl or phenyl,
in which furthermore
$R^3$ represents $C_1$-$C_6$-alkyl, $C_1$-$C_4$-alkoxy-ethyl, allyl, propargyl, 1-methyl-propargyl, 1,1-dimethyl-propargyl, cyanomethyl, cyclopentyl, cyclohexyl, benzyl, naphthyl or phenyl, which is optionally substituted by methyl, chlorine, cyano, nitro or methoxy, multiple and mixed substitution by the indicated radicals also being possible in which furthermore – in the case where n represents zero – the radicals $R^2$ and $R^3$, together with the nitrogen atom to which they are bonded, represent pyrrolidyl, monoalkyl- or dialkyl-pyrrolidyl with 1 to 3 carbon atoms per alkyl group, morpholinyl or dialkylmorpholinyl with 1 to 3 carbon atoms per alkyl group, piperidyl, monoalkyl-, dialkyl- or trialkyl-piperidyl with 1 to 3 carbon atoms per alkyl group, 4,4-dialkoxy-piperidyl with 1 to 3 carbon atoms per alkoxy group, spiro-substituted piperidyl of the formula

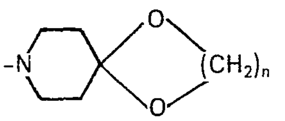

(wherein
n represents 2 or 3),
perhydroazepinyl (hexamethyleneimino radical), trimethyl-perhydroazepinyl, the heptamethyleneimino radical, the dodecamethyleneimino radical, 1,2,3,4-tetrahydroindolyl, monoalkyl-, dialkyl- or trialkyl-1,2,3,4-tetrahydroindolyl with up to 3 carbon atoms per alkyl group, perhydroindolyl, monoalkyl-, dialkyl- or trialkyl-perhydroindolyl with 1 to 3 carbon atoms per alkyl group, 1,2,3,4-tetrahydroquinolyl or 1,2,3,4-tetrahydro-iso-quinolyl, monoalkyl-, dialkyl- or trialkyl-1,2,3,4-tetrahydro-quinolyl or -isoquinolyl with 1 to 3 carbon atoms per alkyl group, perhydro-quinolyl or perhydro-iso-quinolyl, monoalkyl-, dialkyl- or trialkyl-perhydroquinolyl or -perhydroisoquinolyl with 1 to 3 carbon atoms per alkyl group, perhydrothiazolyl, perhydrooxazolyl, perhydrooxazinyl, the radical

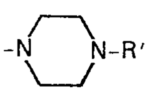

(wherein
R′ represents $C_1$-$C_4$-alkyl, phenyl which is optionally substituted by $C_1$-$C_2$-alkyl, $C_1$-$C_2$-alkoxy, fluorine, chlorine, bromine, trifluoromethyl and/or nitro, or benzyl or phenylethyl) or represents the radical

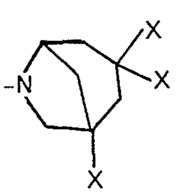

(wherein
X represents hydrogen or methyl), characterised in that α-hydroxycarboxylic acid amides of the formula

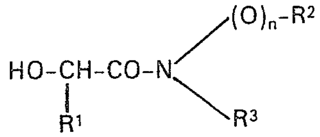 (II)

in which
n, $R^1$, $R^2$ and $R^3$ have the meaning indicated above, are reacted with halogenotetrazoles of the formula

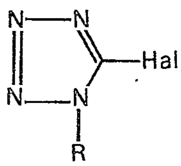 (III)

in which
R has the meaning indicated above and
Hal represents chlorine, bromine or iodine,
if appropriate in the presence of an acid acceptor and if appropriate using a diluent.

3. Herbicidal compositions, characterised in that they contain tetrazolyloxycarboxylic acid amides according to Claim 1.

4. Use of tetrazolyloxycarboxylic acid amides according to Claim 1 for combating plant growth.

5. Process for the preparation of herbicidal agents, characterised in that tetrazolyloxycarboxylic acid amides according to Claim 1 are mixed with extenders and/or surface-active agents.

**Revendications**

1. Amides d'acides tétrazolyloxycarboxyliques de formule:

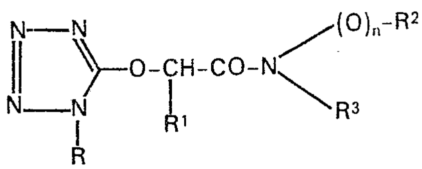 (I)

dans laquelle:
R représente un groupe phényle qui est éventuellement substitué par du chlore, un radical nitro, méthyle et/ou trifluorométhyle, des substitutions multiples et mixtes par les restes mentionnés étant également possibles, en outre
$R^1$ désigne l'hydrogène,
n est égal à 0 ou à 1,
$R^2$ est un groupe alkyle en $C_1$ à $C_6$, (alkoxy en $C_1$ à $C_4$)éthyle, allyle, propargyle, 1-méthylpropargyle ou 1,1-diméthylpropargyle ou bien – lorsque n est égal à 0 –, également un groupe cyanométhyle, cyclopentyle, cyclohexyle, benzyle ou phényle, en outre
$R^3$ est un groupe alkyle en $C_1$ à $C_6$, (alkoxy en $C_1$ à $C_4$)éthyle, allyle, propargyle, 1-méthylpropargyle, 1,1-diméthylpropargyle, cyanométhyle, cyclopentyle, cyclohexyle, benzyle, naphtyle ou phényle qui est éventuellement substitué par un radical méthyle, du chlore, un radical cyano, nitro ou méthoxy, une substitution multiple et mixte par

les restes mentionnés étant également possible, en outre - au cas où n est égal à 0 -, les restes $R^2$ et $R^3$ forment conjointement avec l'atome d'azote auquel ils sont liés, un groupe pyrrolidyle, monoalkyl- ou dialkylpyrrolidyle ayant 1 à 3 atomes de carbone par groupe alkyle, morpholinyle ou dialkylmorpholinyle ayant 1 à 3 atomes de carbone par groupe alkyle, pipéridyle, monoalkyl-, dialkyl- ou trialkylpipéridyle ayant 1 à 3 atomes de carbone par groupe alkyle, 4,4-dialkoxypipéridyle ayant 1 à 3 atomes de carbone par groupe alkoxy, un groupe pipéridyle à substitution spiro de formule:

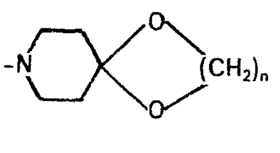

(dans laquelle n est égal à 2 ou à 3), un groupe perhydroazépinyle (reste hexaméthylèneimino), un groupe triméthylperhydroazépinyle, le reste heptaméthylèneimino, le reste dodécaméthylèneimino, un groupe 1,2,3,4-tétrahydroindolyle, monoalkyl-, dialkyl- ou trialkyl-1,2,3,4-tétrahydroindolyle ayant jusqu'à 3 atomes de carbone par groupe alkyle, un groupe perhydroindolyle, monoalkyl-, dialkyl- ou trialkylperhydroindolyle ayant 1 à 3 atomes de carbone par groupe alkyle, un groupe 1,2,3,4-tétrahydroquinolyle ou 1,2,3,4-tétrahydro-isoquinolyle, monoalkyl-, dialkyl- ou trialkyl-1,2,3,4-tétrahydroquinolyle ou -isoquinolyle ayant 1 à 3 atomes de carbone par groupe alkyle, un groupe perhydroquinolyle ou perhydro-isoquinolyle, monoalkyl-, dialkyl- ou trialkylperhydroquinolyle ou -perhydro-isoquinolyle ayant 1 à 3 atomes de carbone par groupe alkyle, un groupe perhydrothiazolyle, un groupe perhydro-oxazolyle, perhydro-oxazinyle, le reste de formule:

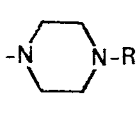

(dans laquelle R' est un groupe alkyle en $C_1$ à $C_4$, un groupe phényle éventuellement substitué par un radical alkyle en $C_1$ ou $C_2$, alkoxy en $C_1$ ou $C_2$, fluoro, chloro, bromo, trifluorométhyle et/ou nitro, un groupe benzyle ou un groupe phényléthyle) ou le reste de formule:

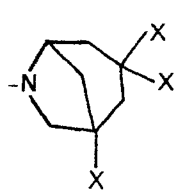

(dans laquelle X désigne l'hydrogène ou le groupe méthyle).

2. Procédé de production d'amides d'acides tétrazolyloxycarboxyliques de formule:

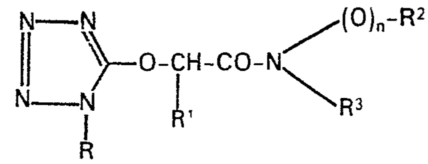

(I)

dans laquelle

R représente un groupe phényle qui est éventuellement substitué par du chlore, un radical nitro, méthyle et/ou trifluorométhyle, des substitutions multiples et mixtes par les restes mentionnés étant également possibles, en outre

$R^1$ désigne l'hydrogène,

n est égal à 0 ou à 1,

$R^2$ est un groupe alkyle en $C_1$ à $C_6$, (alkoxy en $C_1$ à $C_4$)éthyle, allyle, propargyle, 1-méthylpropargyle ou 1,1-diméthylpropargyle ou bien - lorsque n est égal à 0 -, également un groupe cyanométhyle, cyclopentyle, cyclohexyle, benzyle ou phényle, en outre

$R^3$ est un groupe alkyle en $C_1$ à $C_6$, (alkoxy en $C_1$ à $C_4$)éthyle, allyle, propargyle, 1-méthylpropargyle, 1,1-diméthylpropargyle, cyanométhyle, cyclopentyle, cyclohexyle, benzyle, naphtyle ou phényle qui est éventuellement substitué par un radical méthyle, du chlore, un radical cyano, nitro ou méthoxy, une substitution multiple et mixte par les restes mentionnés étant également possible, en outre - au cas où n est égal à 0 -, les restes $R^2$ et $R^3$ forment conjointement avec l'atome d'azote auquel ils sont liés, un groupe pyrrolidyle, monoalkyl- ou dialkylpyrrolidyle ayant 1 à 3 atomes de carbone par groupe alkyle, morpholinyle ou dialkylmorpholinyle ayant 1 à 3 atomes de carbone par groupe alkyle, pipéridyle, monoalkyl-, dialkyl- ou trialkylpipéridyle ayant 1 à 3 atomes de carbone par groupe alkyle, 4,4-dialkoxypipéridyle ayant 1 à 3 atomes de carbone par groupe alkoxy, un groupe pipéridyle à substitution spiro de formule:

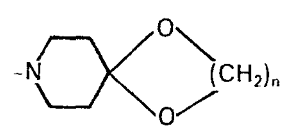

(dans laquelle n est égal à 2 ou à 3), un groupe perhydroazépinyle (reste hexaméthylèneimino), un groupe triméthylperhydroazépinyle, le reste heptaméthylèneimino, le reste dodécaméthylèneimino, un groupe 1,2,3,4-tétrahydroindolyle, monoalkyl-, dialkyl- ou trialkyl-1,2,3,4-tétrahydroindolyle ayant jusqu'à 3 atomes de carbone par groupe alkyle, un groupe perhydroindolyle, monoalkyl-, dialkyl- ou trialkylperhydroindolyle ayant 1 à 3 atomes de carbone par groupe alkyle, un groupe 1,2,3,4-tétrahydroquinolyle ou 1,2,3,4-tétrahydro-isoquinolyle, monoalkyl-, dialkyl- ou trialkyl-1,2,3,4-tétrahydroquinolyle ou -isoquinolyle ayant 1 à 3 atomes de carbone par groupe alkyle, un groupe perhydroquinolyle ou perhydro-isoquinolyle, monoalkyl-, dialkyl- ou trialkylperhydroquinolyle ou -perhydro-isoquinolyle ayant 1 à 3 atomes de carbone par groupe alkyle, un groupe perhydrothiazolyle, un groupe perhydro-oxazolyle, perhydro-oxazinyle, le reste de formule:

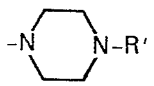

(dans laquelle R′ est un groupe alkyle en $C_1$ à $C_4$, un groupe phényle éventuellement substitué par un radical alkyle en $C_1$ ou $C_2$, alkoxy en $C_1$ ou $C_2$, fluoro, chloro, bromo, trifluorométhyle et/ou nitro, un groupe benzyle ou un groupe phényléthyle) ou le reste de formule:

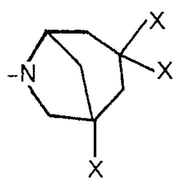

(dans laquelle X désigne l'hydrogène ou le groupe méthyle), caractérisé en ce qu'on fait réagir des amides d'acides α-hydroxycarboxyliques de formule:

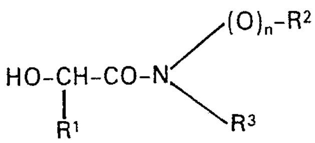

(II)

dans laquelle:
n, $R^1$, $R^2$ et $R^3$ ont la définition indiquée ci-dessus, avec des halogénotétrazoles de formule:

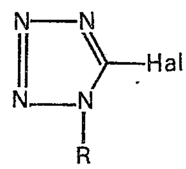

(III)

dans laquelle:
R a la définition indiquée ci-dessus et
Hal désigne du chlore, du brome ou de l'iode, éventuellement en présence d'un accepteur d'acide et en utilisant éventuellement un diluant.

3. Compositions herbicides, caractérisées par une teneur en amides d'acides tétrazolyloxycarboxyliques suivant la revendication 1.

4. Utilisation d'amides d'acides tétrazolyloxycarboxyliques suivant la revendication 1 dans la lutte contre la croissance des plantes.

5. Procédé de production de compositions herbicides, caractérisé en ce qu'on mélange des amides d'acides tétrazolyloxycaboxyliques suivant la revendication 1 avec des diluants et/ou des agents tensio-actifs.